# DEMANDE DE BREVET EUROPEEN

(11) **EP 3 244 180 A1**
(43) Date de publication de la demande: **15.11.2017**
(21) Numéro de dépôt: 17168619.9
(22) Date de dépôt: 28.04.2017
(51) Int. Cl.: G01L 9/00, G01L 19/08

(54) **DISPOSITIF POUR MESURER UNE PRESSION DANS UN FLUIDE ET POMPE EQUIPEE D'UN TEL DISPOSITIF**

(30) Priorité: 10.05.2016 FR 1654151
(71) Demandeur: Abousaleh, Khaled, 8834 Schindellegi (CH)
(72) Inventeur: Abousaleh, Khaled, 8834 Schindellegi (CH)
(74) Mandataire: Mazabraud, Xavier

(57) **Abrégé**

L'invention porte notamment sur un capteur pour mesurer une pression dans un fluide, dont un corps 1 comprend une membrane 2 et une paroi 3 formant un support périphérique pour et autour de la membrane, caractérisé en ce que :
- la membrane et la paroi périphérique sont formées d'une seule pièce ; et,
- la membrane et la paroi périphérique forment ensemble une surface antérieure 4 plane et lisse destinée à être en contact avec ledit fluide.

## Description

La présente invention se rapporte au domaine des capteurs de pression, particulièrement aux capteurs à membrane, notamment de petites tailles.

On connaît de tels capteurs comprenant une membrane rapportée par soudure ou collage sur un boîtier généralement annulaire. Lorsqu'un tel capteur est utilisé pour mesurer la pression dans un liquide, des bulles de gaz viennent se fixer à la jonction entre le boîtier et la membrane. Ceci est d'autant plus préjudiciable à la qualité des mesures que le capteur est petit et que les niveaux de pression à mesurer sont faibles.

De plus, lorsque la membrane est soudée ou collée selon l'art antérieur, il y a un phénomène qui apparaît lorsque la membrane se déforme et que l'on appelle « effet clinquant » ou « oil can effect ». En effet, la déformation de la membrane est non-linéaire lorsque l'on passe d'une pression positive à une pression négative et inversement.

En outre, les différents matériaux utilisés pour réaliser le capteur, notamment les soudures ou les colles, ne sont pas toujours chimiquement et/ou biologiquement compatibles avec le fluide dont on veut mesurer la pression. Ainsi, notamment, de la corrosion peut apparaître à la jonction entre la membrane et le boîtier, ce qui est préjudiciable à la durée de vie du capteur et/ou à la préservation du fluide.

L'invention a pour but de proposer des moyens pour réaliser un capteur qui peut être de petite taille tout en garantissant une durée de vie améliorée et une grande qualité des mesures.

Pour atteindre son but, l'invention propose un corps pour un capteur destiné à la mesure d'une pression dans un fluide, notamment dans un liquide, comprenant une membrane et une paroi formant un support périphérique pour et autour de cette membrane, caractérisé en ce que :
- la membrane et la paroi périphérique sont formées d'une seule pièce ; et,
- la membrane et la paroi périphérique forment ensemble une surface antérieure sensiblement plane et lisse, destinée à être en contact avec le fluide.

De préférence, la paroi périphérique est annulaire et définit, à l'arrière de la membrane, un logement, notamment pour au moins une jauge de contraintes.

L'invention propose aussi un capteur pour mesurer une pression dans un fluide, caractérisé en ce qu'il comprend un corps selon l'invention et au moins une jauge de contrainte, disposée sur une face arrière de la membrane, opposée à la surface antérieure.

L'invention propose aussi un dispositif pour pomper un fluide, notamment une pompe de précision, caractérisé en ce qu'il comprend une chambre de pompage cylindrique autour d'un axe, cette chambre étant axialement fermée par la surface antérieure d'un corps selon l'invention.

L'invention propose encore un dispositif pour mesurer une pression dans un organisme vivant, notamment humain, caractérisé en ce qu'il comprend un capteur selon l'invention, le capteur étant prévu pour être implanté dans cet organisme, ce dispositif comprenant en outre des moyens de lecture sans fil d'une mesure, de préférence de type à radiofréquence, notamment connus sous l'acronyme RFID.

Plusieurs modes d'exécution de l'invention seront décrits ci-après, à titre d'exemples non limitatifs, en référence aux dessins annexés dans lesquels :
- La figure 1 est une vue en perspective du corps d'un capteur selon l'invention ;
- La figure 2 est un vue axiale de l'intérieur du corps de la figure 1 ;
- La figure 3 est une vue en coupe longitudinale du corps, selon le plan III-III de la figure 2 ; et,
- La figure 4 est une vue longitudinale du corps, en position d'usage à l'extrémité d'une chambre de pompage.

Les figures illustrent un corps 1 d'un capteur de pression. Le corps 1 a sensiblement une forme de révolution autour d'un axe X1 ; il est formé d'une seule pièce. Il comprend :
- une paroi antérieure 2, servant de membrane 2 pour le capteur ; et,
- une paroi annulaire 3.

La paroi antérieure, de faible épaisseur E2, est en forme de disque d'axe de révolution X1 et de diamètre membranaire D2. La paroi annulaire constitue une paroi périphérique servant de support à la membrane 2. La membrane 2 et la paroi annulaire 3 forment ensemble une surface antérieure 4 sensiblement plane et lisse. Cette surface antérieure 4 s'étend radialement depuis l'axe X1, en forme de disque de diamètre antérieur D4.

La paroi annulaire 3 définit :
- une première surface intérieure 6 cylindrique, de diamètre D2 ;
- une deuxième surface intérieure 7 cylindrique, de diamètre D7>D2 ;
- une surface annulaire intérieure 8, s'étendant radialement entre les deux surfaces intérieures 6, 7, et formant avec elles un épaulement intérieur ;
- une première surface extérieure 11 cylindrique, de diamètre D4 ;
- une deuxième surface extérieure 12 cylindrique, de diamètre D12 formant le plus grand diamètre du corps, avec D12>D4 ;
- une surface annulaire extérieure 13, s'étendant radialement entre les deux surfaces extérieures 11 et 12, et formant avec elles un épaulement extérieur ; et,
- une surface annulaire postérieure 14, s'étendant radialement entre la deuxième surface intérieure 7 et la deuxième surface extérieure 12.

Les surfaces intérieures définissent ensemble un logement 16. Dans l'exemple illustré, la paroi annulaire 3 est percée d'un trou 17 s'étendant radialement ; ce trou débouche d'une part à travers la première surface intérieure 7 et d'autre part à travers la deuxième surface extérieure 12.

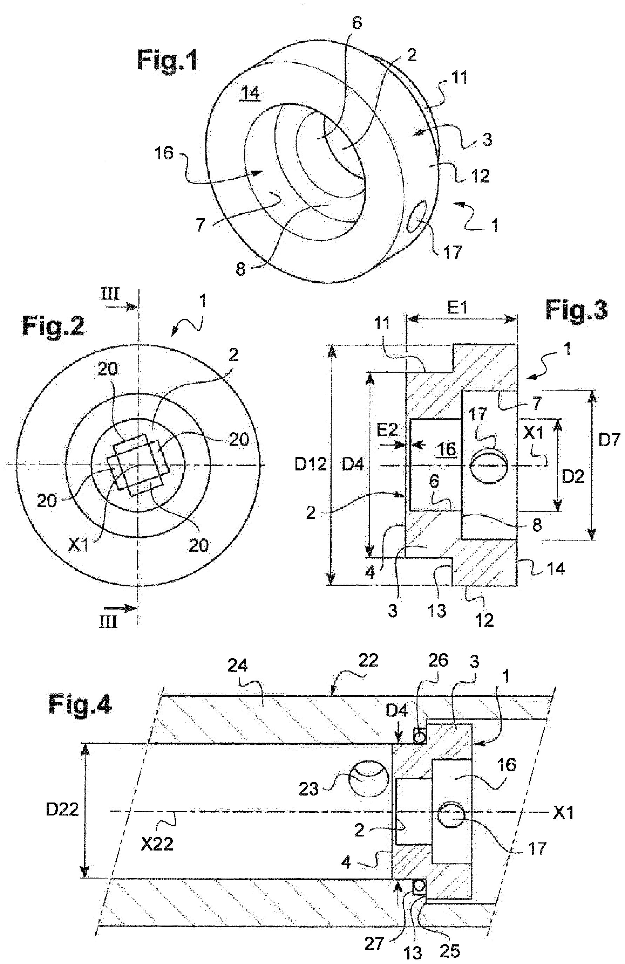

Le logement 16 est notamment prévu pour contenir des jauges de déformation 20, visibles à la figure 1. Le trou 17 permet le passage de fils reliant les jauges 20 avec des moyens, non représentés, pour traiter les déformations de la membrane relevées par les jauges. Dans l'exemple illustré, le capteur comprend quatre jauges 20 fixées à l'arrière de la membrane 2 perpendiculairement l'une à l'autre.

Comme illustré à la figure 4, un capteur selon l'invention peut être utilisé pour mesurer une pression dans une chambre de pompage 22.

Dans l'exemple illustré, la chambre de pompage est cylindrique autour d'un axe X22. Le diamètre D22 de la chambre est sensiblement égal au diamètre antérieur D4 du corps 1. Le corps 1 est introduit à une extrémité axiale de la chambre 22, de sorte que l'axe X1 du corps et l'axe X22 de la chambre sont sensiblement confondus, la surface antérieure 4 constituant ainsi un fond pour la chambre 22, en vis-à-vis d'un piston, non représenté. Un orifice est formé latéralement dans une paroi 24 de la chambre, pour l'admission et/ou l'expulsion du fluide à pomper. Cet orifice est disposé de sorte qu'il vient affleurer avec la surface antérieure 4, c'est-à-dire le fond 4 de la chambre. La surface annulaire extérieure 13 du corps 1 est prévue pour venir en butée axiale contre un premier épaulement 25 de la paroi 24 de la chambre 22 ; un positionnement axial de la paroi antérieure 4 dans la chambre est ainsi assuré. En outre, un joint d'étanchéité 26 torique est monté comprimé entre la paroi annulaire extérieure 13 et un deuxième épaulement 27 de la paroi 24.

L'intérêt de réaliser l'étanchéité contre la surface annulaire extérieure 13 est de diminuer l'influence des contraintes de serrage sur la membrane et les jauges de contraintes. C'est un intérêt majeur de la forme de ce capteur. La plupart des capteurs du commerce réalisent l'étanchéité sur la surface antérieure 4 à côté de la membrane ce qui a pour effet de déformer celle-ci et donc de parasiter la mesure. L'autre avantage de ne pas réaliser l'étanchéité sur la surface antérieure 4 est que cette surface demeure plane et permet la circulation de liquides tangentiellement à la membrane sans piéger du gaz ou des particules.

La membrane 2 a un élancement D2/E2 important. Son épaisseur E2 est faible relativement à l'épaisseur E3= (D4-D2)/2 de la paroi annulaire 3 à l'endroit de la surface antérieure 4, de sorte que la paroi annulaire est rigide relativement à la membrane. Dans l'exemple illustré, les dimensions du capteur sont sensiblement :

| | | |
|---|---|---|
| E1 | = | 3,6 mm |
| E2 | = | 0.06 mm |
| E3 | = | 1.25 mm |
| D2 | = | 3.5 mm |
| D4 | = | 6 mm |
| D12 | = | 8 mm |

De préférence, pour un capteur selon l'invention, on choisit :
0,01 mm < E2 < 1 mm
   et
1 mm < D2 < 5 mm

De préférence, le corps du capteur est en inox ou en Titane ou en un dérivé de l'inox ; il peut être réalisé par usinage ou par impression 3D.

Bien sûr, l'invention n'est pas limitée aux modes de réalisation préférés qui viennent d'être décrits mais, au contraire, l'invention est définie par les revendications qui suivent.

Il apparaîtra en effet à l'homme de l'art que diverses modifications peuvent être apportées aux modes de réalisation décrits ci-dessus, à la lumière de l'enseignement qui vient de lui être divulgué.

Ainsi, au lieu de quatre jauges, le capteur peut en comprendre un nombre différent ; elles peuvent être disposées différemment.

Un capteur selon l'invention est adapté pour équiper une pompe de précision. Il peut aussi équiper un autre dispositif.

Un capteur selon l'invention est particulièrement avantageux, en effet :
- c'est un capteur dont la membrane se déforme, et dont les jauges de contraintes, positionnées à l'intérieur, permettent de mesurer les variations de déplacement de la membrane ;
- le corps est réalisé en une seule pièce sans soudure ni collage ; la matière qui le constitue a, de ce fait, un comportement optimisé car les déformations de la membrane se font dans le domaine élastique de la matière, et la durée de vie du capteur est élevée ;
- l'absence de soudure et de collage permet sa miniaturisation ;
- l'absence de soudure et de collage, éliminant tout risque de porosité, permet une compatibilité chimique et biologique optimale ;
- la membrane affleurante, c'est-à-dire formant une seule et même surface antérieure avec la paroi annulaire, est optimisée pour les liquides car elle ne retient pas les bulles de gaz ; la précision de mesure de la pression en est améliorée et le comportement optimisé du dispositif équipé d'un tel capteur ;
- l'étendue de mesure (en anglais : Full pressure range) et la pression maximale admissible (en anglais : Over pressure) sont élevées, grâce à la résistance élastique de la matière dont est entièrement constitué le corps du capteur ;
- il n'est sensible ni aux vibrations ni aux bruits, seulement aux variations de pression, le rapport entre l'épaisseur de la paroi annulaire du corps et celle de la membrane étant très élevé ;
- par ses caractéristiques dimensionnelles et de compatibilité biologique, ce capteur peut être implantable sans fil dans le corps humain car fiable et a une longue durée de vie ;
- de plus, les jauges utilisées consomment un faible courant, ce qui permet d'avoir un produit implantable ; ce capteur peut même être passif (sans batterie) car les jauges nécessitent une faible énergie d'activation ;
- ce capteur utilise des jauges semi-conductrices miniatures qui ont un facteur de jauge égal à 200, beaucoup plus élevé que le facteur de jauge de jauges métalliques qui est de 3 ; ce qui permet de mesurer de très faibles déformations ;
- avec une membrane usinée selon l'invention, l'effet « clinquant » est corrigé car la linéarité de la déformation est conservée ; cette correction permet de mesurer des pressions plus faibles et de mesurer des faibles variations de pression négatives-positives en ayant une déformation qui reste linéaire.

Un autre intérêt d'un capteur selon l'invention est que les jauges de contraintes peuvent être directement appliquées sur la membrane (isolée électriquement), de sorte que quand il y a déformation de la membrane, il y a une transmission directe de cette déformation sur les jauges ; ceci permet d'avoir une mesure précise et permet de mesurer des faibles déformations. Au contraire, les capteurs du marché contiennent souvent un liquide visqueux ou de l'huile afin de transmettre la déformation de la membrane vers l'élément sensible, ce qui engendre une perte de sensibilité et de précision de la mesure. En outre, des bulles de gaz sont susceptibles d'être présentes dans les liquides utilisés, ce qui engendre un amortissement dans la transmission de la pression et une sensibilité à la température et à la pression ambiante.

## Revendications

1. Corps (1) pour un capteur pour mesurer une pression dans un fluide, comprenant une membrane (2) et une paroi (3) formant un support périphérique pour et autour de ladite membrane, **caractérisé en ce que** :
- ladite membrane et ladite paroi périphérique sont formées d'une seule pièce ; et,
- ladite membrane et ladite paroi périphérique forment ensemble une surface antérieure (4) sensiblement plane et lisse, destinée à être en contact avec ledit fluide.

2. Corps selon la revendication 1, **caractérisé en ce que** la paroi périphérique (3) est annulaire et définit, à l'arrière de la membrane (2), un logement (16), notamment pour au moins une jauge (20) de contraintes.

3. Corps selon l'une des revendications 1 et 2, **caractérisé en ce qu'**il est réalisé d'une seule pièce, par usinage.

4. Corps selon l'une des revendications 1 et 2, **caractérisé en ce qu'**il est réalisé d'une seule pièce, par impression 3D.

5. Corps selon l'une des revendications 1 à 4, **caractérisé en ce que** la membrane (2) a une épaisseur (E2) comprise entre 0,01 mm et 1 mm, et un diamètre (D2) compris entre 1 mm et 5 mm.

6. Capteur pour mesurer une pression dans un fluide, **caractérisé en ce qu'**il comprend un corps (1) selon l'une des revendications précédentes et au moins une jauge de contrainte (20), disposée sur une face arrière de la membrane (2), opposée à la surface antérieure (4).

7. Dispositif pour pomper un fluide, notamment pompe de précision, **caractérisé en ce qu'**il comprend une chambre de pompage cylindrique (22) autour d'un axe (X22), ladite chambre étant axialement fermée par la surface antérieure (4) d'un corps (1) selon l'une des revendications 1 à 5.

8. Dispositif pour mesurer une pression dans un organisme vivant, notamment humain, **caractérisé en ce qu'**il comprend un capteur selon la revendication 6, ledit capteur étant prévu pour être implanté dans ledit organisme, ledit dispositif comprenant en outre des moyens de lecture sans fil d'une mesure, de préférence de type à radiofréquence.
